# EUROPÄISCHE PATENTSCHRIFT

(11) **EP 3 413 122 B1**
(45) Veröffentlichungstag und Bekanntmachung des Hinweises auf die Patenterteilung: **04.03.2020**
(21) Anmeldenummer: 17174925.2
(22) Anmeldetag: 08.06.2017
(51) Int. Cl.: G02C 13/00

(54) **VERFAHREN, VORRICHTUNG UND COMPUTERPROGRAMM ZUM BESTIMMEN EINES NAH-DURCHBLICKPUNKTES**
METHOD, DEVICE AND COMPUTER PROGRAM FOR DETERMINING A CLOSE-UP VIEWPOINT
PROCÉDÉ, DISPOSITIF ET PROGRAMME INFORMATIQUE DESTINÉS À DÉTERMINER UN POINT DE VUE PROCHE

(43) Veröffentlichungstag der Anmeldung: 12.12.2018
(73) Patentinhaber: Carl Zeiss Vision International GmbH, 73430 Aalen (DE)
(72) Erfinder: GAMPERLING, Michael, 89340 Leipheim (DE); ALVAREZ DIEZ, Cristina, 73447 Oberkochen (DE); GLASENAPP, Carsten, 73447 Oberkochen (DE)
(74) Vertreter: Sticht, Andreas

(56) Entgegenhaltungen:
- EP-A1- 2 963 482
- EP-A2- 1 038 495
- WO-A1-2017/064060
- DE-A1-102011 009 646
- DE-A1-102014 200 637
- JP-A- 2003 329 541
- US-A1- 2002 105 530
- US-A1- 2014 293 219
- US-A1- 2016 327 811
- KENNETH ALBERTO FUNES MORA ET AL: "Gaze estimation from multimodal Kinect data", COMPUTER VISION AND PATTERN RECOGNITION WORKSHOPS (CVPRW), 2012 IEEE COMPUTER SOCIETY CONFERENCE ON, IEEE, 16. Juni 2012 (2012-06-16), Seiten 25-30, XP032206804, DOI: 10.1109/CVPRW.2012.6239182 ISBN: 978-1-4673-1611-8

## Beschreibung

Die vorliegende Anmeldung betrifft Verfahren, Vorrichtungen und entsprechende Computerprogramme zum Bestimmen von Nah-Durchblickpunkten von Brillengläsern.

Bei der Einpassung von Brillengläsern in Brillenfassungen werden Zentrierparameter benötigt, um die Lage des Brillenglases in der Brillenfassung angepasst an die jeweilige Person, für die die Brille gedacht ist, einzupassen. Derartige Zentrierparameter sind unter anderem in der DIN EN ISO 13666:2012 definiert. Zu diesen Zentrierparametern zählen Fern-Durchblickpunkte, welche angeben, wo eine Blickrichtung der Person beim Blick in die Ferne durch die Brillengläser hindurchgeht und Nah-Durchblickpunkte, welche angeben, wo eine Blickrichtung der Person bei einem Blick in die Nähe, z.B. beim Lesen, durch die Brillengläser hindurchgeht. Fern-Durchblickpunkt und Nah-Durchblickpunkt sind insbesondere in der DIN EN ISO 13666: 2012 unter 5.16 und 5.17 definiert. Der Nah-Durchblickpunkt wird bei Gleitsichtgläsern benötigt, welche je nach Blickrichtung eine Korrektur für Fern-Sehen und eine Korrektur für Nah-Sehen ermöglichen (vergleiche DIN EN ISO 13666: 2012, 8.3.5). Häufig werden derartige Gleitsichtgläser heute verkauft und in eine Brillenfassung eingebaut, ohne dass die Position der Nah-Durchblickpunkte bestimmt wird, was dazu führen kann, dass der Brillenträger Objekte in der Nähe schlecht sieht.

Um dieses Problem zu beheben, sind verschiedene Ansätze bekannt, um Nah-Durchblickpunkte zu bestimmen.

Die JP 2005-342186 A offenbart beispielsweise ein Verfahren, welches bewegliche Lichtquellen benutzt, die von einer Fernblickrichtung (horizontal von dem Kopf der zu untersuchenden Person) zu einer zu der Horizontalen geneigten Nahblickrichtung wandern. Dabei werden mehrere Bildaufnahmen erstellt. Hier sind die Blickrichtungen durch die beweglichen Lichtquellen vorgegeben.

Die US 2014/009737 A1 offenbart eine relativ komplexe Vorrichtung, um Nah- und Fernzentrierdaten zu bestimmen. Hier werden zwei Kameramodule benutzt, um Bilder einer zu untersuchenden Person für eine Fernblickrichtung und eine Nahblickrichtung aufzunehmen.

Hierzu kann Software auf einem Tabletcomputer installiert werden, und auf einem Bildschirm des Tabletcomputers können Elemente angezeigt werden, die von der zu untersuchenden Person zu betrachten sind. Zudem muss ein Maßstab am Kopf der zu untersuchenden Person angebracht werden.

Die US 2010/149486 A1 misst einen Unterschied einer Kopfvorneigung zwischen einer Fernblickrichtung und einer Leseblickrichtung, wobei die genaue Blickrichtung beim Lesen nicht bestimmt wird, was zu Ungenauigkeiten führen kann.

Die US 2003/123026 A1 bestimmt einen Nah-Pupillenabstand (PD, Pupillary Distance) auf Basis einer Frontaufnahme des Kopfes der zu untersuchenden Person. Die dort verwendete Vorrichtung weist einen kurzen Betrachtungsabstand auf, wodurch es zu einer Verdeckung der Pupillen durch Teile der Brillenfassung kommen kann.

Aus der DE 103 00 188 A1 ist ein Verfahren bekannt, bei welchem Nah-Zentrierparameter mittels Bildaufnahmen bestimmt werden, wobei hier nicht angegeben ist, wie die Bestimmung genau erfolgen soll.

Die meisten der oben beschriebenen Herangehensweisen haben den Nachteil, dass ein Referenzobjekt mit bekannten Abmessungen, beispielsweise ein sogenannter Messbügel, an der Brillenfassung oder am Gesicht angeordnet sein muss, um die Anordnung der Brillenfassung im Raum zu erkennen. Die Verwendung eines derartigen Messbügels stellt jedoch eine nicht unerhebliche Beeinflussung der Position der Fassung im Gesicht der Person dar, und die Person selbst kann durch das Vorhandensein des Messbügels beeinflusst werden, sodass hier Ungenauigkeiten bei der Messung entstehen können. Zudem werden die Nah-Durchblickpunkte bei diesen Verfahren teilweise nur indirekt bestimmt.

Die EP 2 913 704 A1 offenbart eine Vorrichtung, bei welchen Zentrierparameter für eine Fernblickrichtung und eine Nahblickrichtung bestimmt werden können. Die Fernblickrichtung und Nahblickrichtung sind dabei konstruktionsbedingt durch die Vorrichtung vorgegeben. Somit entspricht die durch die Vorrichtung vorgegebene Nahblickrichtung nicht unbedingt der tatsächlichen Nahblickrichtung, welche die zu untersuchende Person beispielsweise beim Lesen einnimmt.

Die WO 2014/061294 A1 offenbart ein Verfahren, bei welchem ein Kopf einer zu untersuchenden Person mit Stroboskoplicht beleuchtet wird und jeweils für eine Fernblickposition und eine Nahblickposition mit einer Kamera aufgenommen wird, welche zwischen Fernblickposition und Nahblickposition bewegbar ist. Die durch das Stroboskoplicht erzeugten Reflexe der Pupillen werden dann ausgewertet. Auch hier entspricht die Nahblickposition nicht notwendigerweise der natürlichen Leseposition der Person, sondern ist durch die Kameraposition bestimmt. Zudem wird eine Stroboskopbeleuchtung benötigt.

Die FR 3 021 205 A1 offenbart eine Herangehensweise zur Bestimmung von Haltungsparametern, bei welchen eine Person bei verschiedenen Tätigkeiten, beispielsweise Lesen, aufgenommen wird, und relative Positionen des Kopfes in den Haltungen auf Basis von übereinstimmenden Punkten bestimmt werden. Eine Bestimmung von Zentrierparametern wie beispielsweise einem Nah-Durchblickpunkt wird in dieser Druckschrift nicht erwähnt.

Die DE 10 2011 009 646 A1 offenbart ein Verfahren zum Bestimmen eines Nah-Durchblickpunktes, bei dem ein Bild eines Kopfes einer Person, während die Person auf ein bewegliches Nahblickziel blickt, aufgenommen wird.

Die Position und/oder Orientierung des Nahblickziels wird bestimmt, und der Nah-Durchblickpunkt (51) wird auf Basis des Bildes und auf Basis der Position und/oder Orientierung des Nahblickziels bestimmt.

Die US 2002/0105530 A1 offenbart das Erzeugen eines 3D-Modells eines Gesichts mittels eines Kamerasystems und einer 3D-Modellierungsanwendung, wobei ein voll texturiertes 3D-Modell des Gesichts erzeugt wird.

Die EP 2 963 482 A1 offenbart ein System zum Linsendesign.

Die europäische Patentanmeldung Nr. 1 038 495 A2 offenbart ein Verfahren gemäß dem Oberbegriff von Anspruch 1 und eine Vorrichtung gemäß dem Oberbegriff von Anspruch 13. Ausgehend von der europäischen Patentanmeldung 1 038 495 A2 ist es eine Aufgabe der vorliegenden Anmeldung, die Bestimmung der Blickrichtung ohne das Verwenden von Messstäben, die an dem Brillengestell oder an der Person angebracht werden müssen, zu ermöglichen.

Hierzu wird nach einem Erfindungsaspekt ein Verfahren gemäß Anspruch 1 und eine Vorrichtung gemäß Anspruch 13 bereitgestellt.

Die DE 10 2014 200 637 A1 offenbart ein Verfahren zum Bestimmen wenigstens eines Anpassparameters für ein in einer Brillenfassung aufnehmbares Brillenglas, bei dem ein in einer Bildebene liegendes Bild wenigstens eines Abschnitts einer einem Proband aufgesetzten Brillenfassung erfasst wird, wobei beim Erfassen des Bilds die Neigung der Bildebene um eine zu der Bildebene parallele horizontale Achse ermittelt wird und bei dem aus dem erfassten Bild des Abschnitts des Probanden mit der dem Probanden aufgesetzten Brillenfassung und der ermittelten Neigung des Bilds der wenigstens eine Anpassparameter ermittelt wird.

Die europäische Anmeldung EP 3 270 099 A1 offenbart ein Verfahren zur Bestimmung eines Hornhaut-Scheitelabstands auf Grundlage von zwei Seitenbildern eines Kopfes einer Person, wobei ein erstes Seitenbild mit aufgesetzter Brille und ein zweites Seitenbild ohne aufgesetzte Brille aufgenommen wird. Aus der europäischen Anmeldung EP 3 270 099 A1 ist jedoch die Verwendung eines 3D-Modells des Kopfes gemäß dem zweiten Erfindungsaspekt nicht bekannt.

Die japanische Patentpublikation Nr. 2003329541 A offenbart Verfahren und Vorrichtung zur Bestimmung von Brillenparametern beim Blick auf ein Nahziel, beispielsweise einer Zeitung die auf eine Acrylplatte geklebt ist, wobei eine Kamera durch ein Loch in der Zeitung die Person, deren Brillenparameter bestimmt werden sollen, aufnimmt.

Die US 2014/0293219 A1 offenbart ein Verfahren zum Bestimmen des Leseabstands einer Person.

Die US 2016/011437 A1 offenbart ein System zum Linsendesign, welches ein Mittel zum Bestimmen eines Augendrehpunktes umfasst, um Sichtlinieninformation zu erhalten. Es werden Verfahren offenbart, die eine Kamera zusammen mit einer "Distance Imaging Photographing Unit" verwenden, um jedem Pixel des Kamerabildes eine Tiefeninformation zuzuordnen. Aus diesen Informationen können dann die Sichtlinieninformationen ermittelt werden.

Ausgehend beispielsweise von der EP 2 913 704 A1 oder auch anderen der genannten Druckschriften ist es eine weitere Aufgabe der vorliegenden Anmeldung, ein Verfahren, eine Vorrichtung und ein Computerprogramm zur Bestimmung eines Nah-Durchblickpunktes bereitzustellen, bei welchen eine zu untersuchende Person die Blickrichtung bei Bestimmung des Nah-Durchblickpunkts möglichst frei wählen kann und bei welchen kein separater Messbügel benötigt wird.

Hierfür werden ein Verfahren nach Anspruch 1, ein Computerprogramm nach Anspruch 12 und eine Vorrichtung nach Anspruch 13 bereitgestellt. Die Unteransprüche definieren weitere Ausführungsformen.

Erfindungsgemäß wird ein Verfahren zum Bestimmen eines Nah-Durchblickpunktes bereitgestellt. Das Verfahren umfasst ein Aufnehmen eines Bildes eines Kopfes mit Augen einer Person, wobei das Aufnehmen des Bildes mit einer in einem Nahblickziel eingebauten Kamera erfolgt, während die Person auf das Nahblickziel blickt, wobei das Bild eine Pupillenposition von Augen zeigt, wobei das Nahblickziel beweglich ist.

Das Verfahren umfasst dabei weiter:
Bestimmen einer Orientierung des Nahblickziels,
Bestimmen einer Blickrichtung der Person beim Blick auf das Nahblickziel auf Basis des Bildes.

Das Verfahren ist dadurch gekennzeichnet, dass das Bestimmen des Nah-Durchblickpunktes auf Basis des Bildes, auf Basis der Blickrichtung und auf Basis einer Lage einer Brillenfassung erfolgt, wobei das Bestimmen der Blickrichtung umfasst:
Ein Bestimmen einer Position einer Pupille eines Auges der Augen in dem Bild, Bestimmen einer Richtung der Pupille relativ zu einer Symmetrieachse der Kamera basierend auf einem Bildwinkel, einer Auflösung der Kamera und der Position der Pupille des einen Auges in dem Bild, Bestimmen einer Richtung der Symmetrieachse basierend auf der Orientierung des Nahblickziels , Bestimmen der Blickrichtung basierend auf der Richtung der Pupille und der Richtung der Symmetrieachse. Hierbei basiert das Bestimmen des Nah-Durchblickpunktes ferner auf einer der folgenden Alternativen:
i) wenn die Person eine Brillenfassung trägt:
   Identifizieren und Bestimmen einer Lage der Brillenfassung in dem aufgenommenen Bild, wobei das Bestimmen des Nah-Durchblickpunktes ferner auf der Lage der Brillenfassung basiert,
ii) wenn die Person keine Brillenfassung trägt:
   Bestimmen des Nah-Durchblickpunktes auf Basis der bestimmten Blickrichtung und eines Modells des Kopfes, an welchen ein Modell einer Brillenfassung angepasst ist.

Zu beachten ist, dass der Bezug auf "einen Nah-Durchblickpunkt" hier bedeutet, dass der Nah-Durchblickpunkt für ein Auge oder für beide Augen der Person bestimmt werden können.

Unter der Position des Nahblickziels ist die Position im dreidimensionalen Raum zu verstehen, wie sie beispielsweise in kartesischen Koordinaten bezüglich eines raumfesten Koordinatensystems angegeben werden kann. Die Orientierung des Nahblickziels beschreibt, wie das Nahblickziel an seiner Position ausgerichtet ist, beispielsweise in welche Richtung eine Seite des Nahblickziels zeigt, auf welche die Person zur Messung blicken soll. Die Kombination von Position und Orientierung wird nach der DIN EN ISO 8373: 2012-03 auch als Pose bezeichnet und spielt insbesondere auch in der Robotertechnik eine große Rolle.

Ein Nahblickziel ist ein Gegenstand, auf welches die Person zur Bestimmung des Nah-Durchblickpunktes blicken soll, wobei beispielweise eine Lesehaltung eingenommen werden kann. Hierzu kann das Nahblickziel einen zu lesenden Text oder ein zu betrachtendes Bild aufweisen, welches die Person in einer normalen Lesehaltung betrachten soll. Dadurch, dass das Nahblickziel beweglich ist, kann eine natürliche Lesehaltung eingenommen werden, und die Messung ist nicht auf eine von der Vorrichtung vorgegebene Lesehaltung beschränkt. Durch die Bestimmung auf Basis des Bildes und der Position und/oder Orientierung des Nahblickziels kann zudem auf die Verwendung eines Messbügels oder ähnlichen Gegenstands verzichtet werden.

Das Bestimmen des Nah-Durchblickpunktes umfasst ein Bestimmen einer Blickrichtung der Person beim Blicken auf das Nahblickziel auf Basis des Bilds umfasst, wobei das Bestimmen des Nah-Durchblickpunktes auf Basis der Blickrichtung erfolgt. Mittels der Blickrichtung kann der Nah-Durchblickpunkt auf einfache Weise ermittelt werden

Hierzu umfasst das Verfahren bevorzugt weiterhin ein Bestimmen einer relativen Lage einer von der Person getragenen Brillenfassung zu einem Augendrehpunkt der Person.

So kann der Nah-Durchblickpunkt einfach bestimmt werden, indem ausgehend von dem Augendrehpunkt ein Schnittpunkt der Blickrichtung mit einem Brillenglas, dessen Lage durch die Lage der Brillenfassung definiert ist, ermittelt wird. Zudem wird so lediglich die Blickrichtung benötigt, jedoch nicht der Abstand zwischen der Person und dem Nahblickziel, der beliebig gewählt werden kann. Unter einer Brillenfassung ist dabei in Übereinstimmung mit DIN EN ISO 7998 und DIN EN ISO 8624 ein Gestell oder eine Halterung zu verstehen, mittels dem/der Brillengläser am Kopf getragen werden können. Der Begriff wie hier verwendet beinhaltet insbesondere auch randlose Brillenfassungen.

Das Bestimmen der relativen Lage der von der Person getragenen Brillenfassung zu dem Augendrehpunkt der Person kann mittels weiterer Bildaufnahmen erfolgen, beispielsweise im Zuge von Bildaufnahmen, welche zur Bestimmung des Fern-Durchblickpunktes erstellt werden. Entsprechende Vorrichtungen, welche Zentrierparameter wie den Fern-Durchblickpunkt auf Basis mehrere Bildaufnahmen bestimmen, sind in den europäischen Patentanmeldungen Nr. 17 153 559.4 sowie Nr. 17 153 556.0 beschrieben.

So kann mithilfe von Parametern wie der relativen Lage der von der Person getragenen Brillenfassung zu dem Augendrehpunkt der Person, die von einer vorherigen Bestimmung des Fern-Durchblickpunktes ohnehin vorliegen, ein Nah-Durchblickpunkt einfach bestimmt werden.

Aus Fern- und Nah-Durchblickpunkt kann dann zusätzlich die sogenannte Progressionskanallänge (siehe 14.1.25 in DIN EN ISO 13666:2013-10) bestimmt werden, die den Abstand zwischen Nah-Durchblickpunkt und Fern-Durchblickpunkt angibt. Zudem oder alternativ kann der nasale Abstand der Nah-Durchblickpunkte relativ zu den Fern-Durchblickpunkten bestimmt werden. Dieser nasale Abstand wird manchmal als "Inset" bezeichnet, stimmt aber nicht notwendigerweise mit dem zwischenzeitlich in der DIN EN ISO 13666:2013-10 unter 14.2.13 normierten Inset überein. Herkömmlicherweise ist dieser nasale Abstand durch das Glasdesign des Gleitsicht-Brillenglases oder des Bifokal-Brillenglases fest vorgegeben. Dadurch wird nicht berücksichtigt, dass dieser nasale Abstand beider Seiten ggf. asymmetrisch sein kann, z.B. dann, wenn der Proband den Kopf normalerweise leicht seitlich geneigt hält, der Blick zum Lesen aber senkrecht nach unten schwenkt, ohne die Seitenneigung des Kopfes zu verändern. Eine derartige Asymmetrie kann durch die Bestimmung dieses nasalen Abstands auf Basis der wie oben beschrieben bestimmten Nah-Durchblickpunkte und Fern-Durchblickpunkte ermittelt werden.

Bei einer Variante erfolgen dabei das Aufnehmen des Bildes als Aufnahme mehrerer Bilder mit der gleichen Kameraeinrichtung, mittels derer die weiteren Bildaufnahmen erstellt werden. Die in den oben genannten europäischen Patentanmeldungen Nr. 17 153 559.4 sowie Nr. 17 153 556.0 beschriebene Kameraeinrichtung weist hierfür mehrere Kameras auf, welche in einem Winkel von etwa 90° zueinander angeordnet sind, um Front- und Seitenaufnahmen zu erstellen. In so aufgenommenen mehreren Bildern können dann mittels der in den obigen Anmeldungen beschriebenen Algorithmen eine Lage der Brillenfassung und eine Augenposition bestimmt werden. Diese Bestimmung kann auf Basis einer Bestimmung der Lage der Brillenfassung in den weiteren Bildaufnahmen vorgenommen werden, d. h. die dort bestimmte Form der Brillenfassung kann mittels Bildanalyse-Algorithmen wie beispielsweise in der europäischen Patentanmeldung Nr. 17153651.9 beschrieben in dem aufgenommenen Bild bestimmt werden, oder es kann eine gemeinsame Bestimmung durchgeführt werden. Bei den in der europäischen Patentanmeldung Nr. 17153651.9 beschriebenen Algorithmen werden insbesondere Lagen von Glasrändern von Brillengläsern detektiert, welche Fassungsrändern der Brillenfassung entsprechen. Hierdurch ist die Lage der Brillenfassung, insbesondere die Lage der Fassungsebene (DIN EN ISO 13666:2012; 17.2), ebenfalls bekannt. Eine Bestimmung einander entsprechender Elemente, in diesem Fall der Brillenfassungen, in den mehreren Bildern sowie in den weiteren Bildaufnahmen kann auch wie in der US 6,944,320 B2 oder der US 7,149,330 B2 beschrieben erfolgen. Auf diese Weise ist eine robuste Bestimmung der Lage der Brillenfassung möglich.

Mit der Lage der Brillenfassung, insbesondere der Fassungsebene, kann dann wie oben beschrieben der Nah-Durchblickpunkt bestimmt werden.

Alternativ kann die Bestimmung der Lage der Brillenfassung auch gemeinsam in dem aufgenommenen Bild und den weitere Bildaufnahmen erfolgen. Eine gemeinsame Verarbeitung des Bilds und der weiteren Bildaufnahme zur Bestimmung der Brillenfassungen, insbesondere der Fassungsränder und somit der Brillengläser, erhöht die Robustheit des Verfahrens weiter.

Bei einer derartigen Herangehensweise mit einer Kameraeinrichtung kann die Position des Nahblickziels im Raum beispielsweise durch Objektverfolgung mittels weiterer Kameras oder durch eine mechanische mit Sensoren versehene Verbindung zu der Kameravorrichtung erfasst werden. Eine solche Objektverfolgung ist z.B. in T. Dedek, "Entwurf und Implementierung einer Objektverfolgung unter Verwendung einer Smart-Camera mit PTZ-Funktionalität", Bachelorarbeit 2009, https://www.ipi.uni-hannover.de/fileadmin/institut/pdf/Abschlussarbeiten/Bachelorarbeit_Dedek_2009.pdf, beschrieben.

Die Blickrichtung kann in diesem Fall bestimmt werden, indem auf Basis des Bildes Pupillenpositionen in drei Dimensionen bestimmt werden und die Blickrichtung dann als Differenz zwischen der Position des Nahblickziels und der Position der Pupillen bestimmt werden. Die Position der Pupillen kann dabei mit den in den oben genannten Anmeldungen bestimmten Verfahren bestimmt werden.

Der oben erwähnte Augendrehpunkt, Englisch: "Center of Rotation" (COR), kann mithilfe bekannter empirischer Daten (siehe beispielsweise Bennett, A., Rabbetts, R.: Clinical Visual Optics, Third Edition, Oxford (1998), S143/144) oder unter Berücksichtung einer refraktionsabhängigen Lage hinter der Pupille bestimmt werden und somit dessen Position relativ zu der Lage der Brillenfassung und somit den Fassungsrändern angegeben werden. Auf diese Weise ist die Bestimmung mittels herkömmlicher Verfahren möglich.

Bei einer anderen Ausführungsform erfolgt das Aufnehmen des Bildes durch eine in das Nahblickziel eingebaute Kamera, und die Orientierung des Nahblickziels und somit der Kamera wird durch Neigungssensoren in dem Nahblickziel bestimmt. Die Pupillenposition der Augen wird dann in dem aufgenommenen Bild bestimmt. Bei bekanntem Bildwinkel und Auflösung der Kamera, welche sich aus den Spezifikationen der Kamera ergeben, kann für die Pupillen eine Richtung relativ zu einer Symmetrieachse der Kamera (die üblicherweise der optischen Achse der Kamera entspricht) angegeben werden. Die Richtung dieser Symmetrieachse ergibt sich aus der ermittelten Neigung des Nahblickziels. Somit kann bei dieser Ausführungsform die Blickrichtung beider Augen direkt bestimmt werden, und wie oben erläutert auf Basis der Blickrichtung auf das Nahblickziel ein Nah-Durchblickpunkt für jedes Auge.

Die Kamera des Nahblickziels kann intrinsisch kalibriert sein, um Bildfeldverzeichnungen zu korrigieren. Intrinsische Kalibrierung bedeutet, dass die Bildfeldverzeichnung gemessen wird und dann korrigiert wird. Dies erhöht die Genauigkeit der Bestimmung des Nah-Durchblickpunktes. Falls die Genauigkeit der Bestimmung auch ohne eine solche Kalibrierung ausreichend ist, beispielsweise weil die Verzeichnungen kleiner als 10 % sind, kann die intrinsische Kalibrierung der Kamera des Nahblickziels auch entfallen.

Bevorzugt wird in einem derartigen Fall als Nahblickziel ein Tablet-Computer oder Smartphone verwendet. Derartige Geräte verfügen mit der Frontkamera über eine geeignete Kamera, weisen eingebaute Neigungssensoren und eine Anzeige zum Anzeigen beispielsweise von einem zu lesenden Text oder eines zu betrachtenden Bildes auf, sodass hier durch einfache Programmierung das erfindungsgemäße Verfahren implementiert werden kann. Das aufgenommene Bild und die gemessenen Neigungen können dann direkt in dem Tablet oder Smartphone ausgewertet werden oder mittels einer Schnittstelle, wie sie ebenfalls üblicherweise vorhanden ist, drahtgebunden oder drahtlos an eine Recheneinrichtung zur weiteren Auswertung übersendet werden.

Das Verfahren kann zudem eine Korrektur der bestimmten Blickrichtungen und/oder des ermittelten Nah-Durchblickpunktes umfassen, sodass ein Bediener wie ein Optiker diese Größen, d.h. die Blickrichtungen und/oder den Nah-Durchblickpunktes vornehmen kann, zum Beispiel falls er der Meinung ist, dass die zu untersuchende Person bei dem Aufnehmen des Bildes nicht die korrekte Kopfhaltung eingenommen hat. Auf diese Weise können also Ungenauigkeiten, die sich aus einer derarigen nicht korrekten Kopfhaltung ergeben, noch korrigiert werden.
Bei manchen der oben beschriebenen Verfahren wird wie beschrieben die Brillenfassung in dem aufgenommenen Bild identifiziert und die so bestimmte Lage der Brillenfassung zur Bestimmung des Nah-Durchblickpunktes herangezogen. Hierfür trägt die Person bei der Aufnahme des Bildes die Brillenfassung.

Bei anderen Ausführungsformen wird ein virtuelles Modell, insbesondere ein 3D-Modell, des Kopfes der Person verwendet, wobei an dieses 3D-Modell ein Modell einer Brillenfassung angepasst ist. Auf Basis dieser Modelle (Modell des Kopfes mit angepasstem Modell der Brillenfassung) und der wie oben beschrieben ermittelten Blickrichtung kann dann wiederum der Nah-Durchblickpunkt bestimmt werden, da die Lage der Brillenglasebenen und die Augendrehpunkte dem 3D-Modell des Kopfes mit angepasstem Modell der Brillenfassung entnehmbar sind. Ein Vorteil der Verwendung von Modellen ist, dass schnell zwischen verschiedenen Brillenfassungen gewechselt werden kann, ohne dass erneut eine Bildaufnahme erfolgen muss. Dabei wird angenommen, dass die Blickrichtung beim Blick auf das Nahblickziel unabhängig von der getragenen Brillenfassung ist.

Unter einem Modell, insbesondere 3D-Modell, ist eine Repräsentation, im Falle eines 3D-Modells eine dreidimensionale Repräsentation von realen Objekten zu verstehen, die als Datensatz in einem Speichermedium, beispielsweise einem Speicher eines Computers oder einem Datenträger, vorliegen. Eine solche dreidimensionale Repräsentation kann beispielsweise ein 3D-Netz (englisch "3D mesh"), bestehend aus einem Satz von 3D-Punkten, die auch als Vertices bezeichnet werden, und Verbindungen zwischen den Punkten, die auch als Edges bezeichnet werden. Diese Verbindung bilden im einfachsten Fall ein Dreiecksnetz (englisch triangle mesh). Bei einer derartigen Repräsentation als 3D-Netz wird nur die Oberfläche eines Objekts beschrieben, nicht das Volumen. Das Netz muss nicht notwendigerweise geschlossen sein. Wird also beispielsweise der Kopf in Form eines Netzes beschrieben, so erscheint er wie eine Maske. Näheres zu derartigen 3D-Modellen findet sich in Rau J-Y, Yeh P-C. "A Semi-Automatic Image-Based Close Range 3D Modeling Pipeline Using a Multi-Camera Configuration." Sensors (Basel, Switzerland). 2012;12(8):11271-11293. doi:10.3390/s120811271; insbesondere Seite 11289, Abbildung "Fig.16".)

Eine weitere Möglichkeit der Repräsentation eines 3D-Modells ist ein Voxel-Gitter (englisch "voxel grid"), welches eine volumenhafte Repräsentation darstellt. Dabei wird der Raum in kleine Würfel oder Quader eingeteilt, welche als Voxel bezeichnet werden. Für jedes Voxel wird im einfachsten Fall die An- oder Abwesenheit des darzustellenden Objekts in Form eines Binärwerts (1 oder 0) gespeichert. Bei einer Kantenlänge der Voxel von 1 mm und einem Volumen von 300 mm x 300 mm x 300 mm, was ein typisches Volumen für einen Kopf darstellt, erhält man somit insgesamt 27 Millionen derartiger Voxel. Derartige Voxel-Gitter sind beispielsweise in M. Nießner, M. Zollhöfer, S. Izadi, and M. Stamminger. "Real-time 3D reconstruction at scale using voxel hashing". ACM Trans. Graph. 32, 6, Article 169 (November 2013),. DOI: https://doi.org/10.1145/2508363.2508374 beschrieben.

Die Brillenglasebene bezeichnet eine Ebene, welche die Lage eines jeweiligen Brillenglases (das im Allgemeinen gekrümmt ist) annähert. Sie kann insbesondere eine Ebene sein, die eine geringste Abweichung (beispielsweise nach dem Kriterium der kleinsten Quadrate) von dem Fassungsrand des jeweiligen Brillenglases aufweist. Der Fassungsrand kann wie in der EP 17153651.9 beschrieben identifiziert werden. Alternativ kann als Brillenglasebene die Scheibenebene gemäß DIN EN ISO 13666:2012; 17.1 verwendet werden. Die Bestimmung dieser Scheibenebene setzt jedoch wie in der DIN EN ISO 13666:2012 die Kenntnis der Form einer Stützscheibe voraus, während bei der obigen Definition lediglich die Lage des Fassungsrandes benötigt wird.

Die Anpassung des Modells der Brillenfassung an das Modell des Kopfes kann dabei wie in der US 2003/0123026 A1, der US 2002/105530 A1, der US 2016/0327811 A1 oder der europäischen Patentanmeldung Nr. 17173929.5 beschrieben erfolgen.

Bei einer Ausführungsform wird die Änderung der Kopfhaltung beim Blick auf das Nahblickziel relativ zur Kopfhaltung beim Blick in die Ferne bestimmt. Dies kann mittels einem oder mehrerer Bilder wie oben beschrieben erfolgen, wobei in diesem Fall bei der Aufnahme des einen oder der mehreren Bilder von der Person keine Brille getragen werden muss. Das 3D-Modell des Kopfes kann dann entsprechend der Änderung der Kopfhaltung geneigt werden oder im Falle von mehreren Kameras auf Basis der Bilder erstellt werden. Durch Identifizierung einander entsprechender Merkmale in den aufgenommen Bildern kann dann unter Berücksichtigung der bekannten Aufnahmepositionen das Modell erstellt werden (siehe z.B. H. Hirschmuller, "Stereo Processing by Semiglobal Matching and Mutual Information," in IEEE Transactions on Pattern Analysis and Machine Intelligence, vol. 30, no. 2, pp. 328-341, Feb. 2008. doi: 10.1109/TPAMI.2007.1166). Mit der wie oben beschrieben bestimmten Blickrichtung beim Blick auf das Nahblickziel ist dann nach dem virtuellen Anpassen des Modells der Brillenfassung bekannt, wo die Fern- und Nah-Durchblickpunkte liegen, da z.B. damit die relative Lage von Augendrehpunkt und Brillenglasebenen entsprechend den Fassungsrändern aus den Modellen bekannt sind.

Bei einer anderen Ausführungsform werden die Positionen der Pupillen im Raum bei Fernblickrichtung und Nahblickrichtung mit Hilfe von Bildaufnahmen bestimmt. Zusammen mit der Position eines weiteren Gesichtspunktes kann dann das Modell des Kopfes in eine Position entsprechend der Kopfhaltung der Person beim Blick auf das Nahblickziel gebracht werden. Als weiterer Gesichtspunkt kann die Nasenspitze oder ein Punkt an den Ohren herangezogen werden, da die relative Lage dieser Punkte zu den Pupillen sich bei Bewegungen kaum ändert. Ansonsten erfolgt die Bestimmung des Nah-Durchblickpunktes wie oben beschrieben.

Alternativ kann die Veränderung der Kopfhaltung beim Blick auf das Nahblickziel auch nur auf Basis von Seitenbildern des Kopfes, die beim Blick in die Ferne und beim Blick auf das Nahblickziel aufgenommen werden, erfolgen. Durch Identifizieren entsprechender Merkmale in den Seitenbildern (z.B. wie in der obigen Referenz Hirschmüller et al. beschrieben) kann dann die Veränderung der Kopfneigung bestimmt und auf das 3D-Modell übertragen werden.

Seitenbilder des Kopfes sind Bilder, auf welchen der Kopf von der Seite, im Wesentlichen in Richtung einer Schläfenpartie des Kopfes, sichtbar ist. In anderen Worten erfolgt die Bildaufnahme bei Seitenbildern im Wesentlichen in einer Richtung, die einer Verbindungslinie zwischen den Ohren des Kopfes entspricht. In einem Seitenbild ist nur ein Auge der Person sichtbar.

Frontbilder sind demgegenüber Bilder, bei denen die Bildaufnahme im Wesentlichen senkrecht zu einer Ebene, welche durch die Augen und den Mund definiert ist, erfolgt. Hier sind im Regelfall beide Augen, die Nase und der Mund abgebildet.

Somit gibt es verschiedene Möglichkeiten, die Lage des Nah-Durchblickpunktes auf Basis von Modellen von Kopf und Brillenfassung zu bestimmen.

Die Verwendung einer (realen) Brillenfassung bei den Bildaufnahmen kann auch mit der Verwendung von Modellen von Kopf und Brillenfassung kombiniert werden. Hierbei wird der Nah-Durchblickpunkt zunächst wie beschrieben für die reale Brillenfassung bestimmt. Ergebnisse dieser Bestimmung wie Kopfhaltung und Blickrichtung können dann auf die Verwendung der Modelle übertragen werden. Somit können die Ergebnisse für den Nah-Durchblickpunkt auf andere Brillenfassungen mittels Modellen derselben übertragen werden, ohne dass erneut Bilder aufgenommen werden müssen.

Die Position und/oder Orientierung des Nahblickziels kann auch manuell (beispielsweise mit einem Meterstab) bestimmt werden und dann manuell eingegeben werden. Die Position der Pupillen kann auch geschätzt werden.

Gemäß einer weiteren Ausführungsform wird ein Computerprogramm mit einem Programmcode bereitgestellt, welches, wenn es auf einer Recheneinrichtung ausgeführt wird, bewirkt, dass eines der oben beschriebenen Verfahren bereitgestellt wird.

Gemäß einem weiteren Aspekt wird eine Vorrichtung zum Bestimmen eines Nah-Durchblickpunktes bereitgestellt, umfassend:
- eine Recheneinrichtung,
- ein Nahblickziel, welches beweglich ist und eine Bildaufnahmeeinrichtung und eine Positionserfassungseinrichtung umfasst, wobei die Bildaufnahmeeinrichtung eingerichtet ist, ein Bild eines Kopfes mit Augen einer Person aufzunehmen, während die Person auf das Nahblickziel blickt. Ferner ist die Positionserfassungseinrichtung zum Bestimmen einer Orientierung des Nahblickziels eingerichtet und die Recheneinrichtung ist zum Bestimmen einer Blickrichtung der Person beim Blick auf das Nahblickziel auf Basis des Bildes eingerichtet.

Die Vorrichtung ist dadurch gekennzeichnet, dass:
- die Recheneinrichtung weiter eingerichtet ist zum Bestimmen des Nah-Durchblickpunktes auf Basis des Bildes, auf Basis einer Lage einer Brillenfassung und auf Basis der Blickrichtung, wobei das Bestimmen der Blickrichtung umfasst:
- ein Bestimmen einer Position einer Pupille eines Auges der Augen in dem Bild,
- Bestimmen einer Richtung der Pupille relativ zu einer Symmetrieachse der Kamera basierend auf einem Bildwinkel, einer Auflösung der Kamera und der Position der Pupille des einen Auges in dem Bild,
- Bestimmen einer Richtung der Symmetrieachse basierend auf der Orientierung des Nahblickziels .
- Bestimmen der Blickrichtung basierend auf der Richtung der Pupille und der Richtung der Symmetrieachse. Hierbei basiert das Bestimmen des Nah-Durchblickpunktes ferner auf einer der folgenden Alternativen:
   i) wenn die Person eine Brillenfassung trägt:
      Identifizieren und Bestimmen einer Lage der Brillenfassung in dem aufgenommenen Bild, wobei das Bestimmen des Nah-Durchblickpunktes ferner auf der Lage der Brillenfassung basiert,
   ii) wenn die Person keine Brillenfassung trägt:
      Bestimmen des Nah-Durchblickpunktes auf Basis der bestimmten Blickrichtung und eines Modells des Kopfes, an welchen ein Modell einer Brillenfassung angepasst ist.

Die Vorrichtung kann zur Durchführung einer der oben beschriebenen Verfahren eingerichtet sein. Insbesondere kann hierzu die Bildaufnahmeeinrichtung wie oben beschrieben zwei oder mehr Kameras umfassen, mit welchen ein Frontbild und ein oder mehrere Seitenbilder gleichzeitig aufnehmbar sind. Auch kann das Nahblickziel die Kameraeinrichtung und/oder Lagesensoren als Positionserfassungseinrichtung umfassen, wie oben beschrieben. Die Positionserfassungseinrichtung kann wie ebenfalls beschrieben mechanische Sensoren oder ein oder mehrere Kameras umfassen. Das ober erwähnte Computerprogramm kann in der Vorrichtung gespeichert sein, um durch die Recheneinrichtung ausgeführt zu werden.

Im Folgenden werden Ausführungsbeipsiele der vorliegenden Erfindung unter Bezugnahme auf die beiliegenden Zeichnungsfiguren näher erläutert. Es zeigen:
Fig. 1 eine Vorrichtung gemäß den Stand der Technikzur Bestimmung eines Fern-Durchblickpunktes, auf Basis derer die Erfindungs implementiert sein kann.,
Fig. 2 eine Draufsicht auf die Vorrichtung der Fig. 1,
Figuren 3A und 3B erläuternde Ansichten zur Bestimmung eines Fern-Durchblickpunktes,
Fig. 4 eine Erweiterung der Vorrichtung der Fig. 1 für eine Bestimmung eines Nah-Durchblickpunktes gemäß einem Ausführungsbeispiel der vorliegenden Erfindung,
Figuren 5A und 5B Diagramme zur Veranschaulichung der Bestimmung von Nah-Durchblickpunkten gemäß Ausführungsbeispielen der Erfindung,
Fig. 6 eine schematische Ansicht eines Nahblickziels gemäß einem Ausführungsbeispiel, und
Fig. 7 ein Flussdiagramm eines Verfahrens gemäß einem Ausführungsbeispiel.

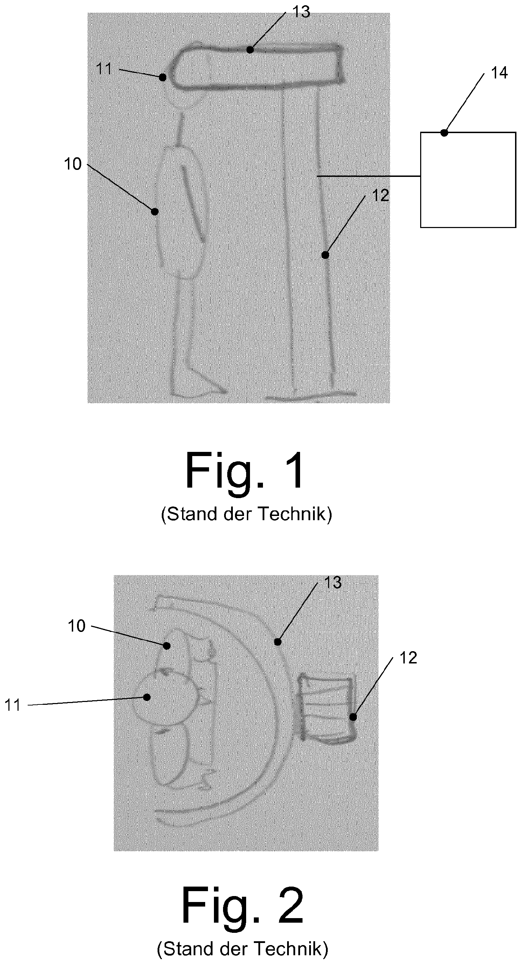

Die Fig. 1 zeigt eine Vorrichtung, wie sie in den bereits erwähnten europäischen Patentanmeldungen 17 153 559.4 und 17 153 556.0 näher beschrieben ist, in einer Seitenansicht. Auf Basis dieser Vorrichtung kann als Erweiterung die vorliegende Erfindung implementiert werden. Die Fig. 2 zeigt eine Draufsicht auf die Vorrichtung der Fig. 1. Die Vorrichtung umfasst eine Kameraeinrichtung 13, welche auf einer Säule 12 angebracht ist. Die Kameraeinrichtung 13 umfasst mehrere Kameras, unter anderem eine Frontkamera zum Aufnehmen eines Frontbildes eines Kopfes 11 einer zu untersuchenden Person 10 sowie Seitenkameras zum Aufnehmen von Seitenbildern des Kopfes 11. In den Figuren 1 und 2 hat die Person 10 eine Kopfhaltung zur Bestimmung eines Fern-Durchblickpunktes, d. h. sie blickt im Wesentlichen horizontal geradeaus auf ein in der Vorrichtung bereitgestelltes Ziel. Eine Recheneinrichtung 14 ist mit der Vorrichtung zur Auswertung der aufgenommenen Bilder verbunden, insbesondere um die Fern-Durchblickpunkte zu bestimmen.

Wie in den oben genannten europäischen Patentanmeldungen dabei näher erläutert, werden im Zuge der Bestimmung des Fern-Blickpunktes eine Lage einer Brillenfassung, welche die Person 10 an dem Kopf 11 trägt, sowie Lagen der Pupillen der Person bestimmt. Die Lage der Brillenfassung oder zumindest von Fassungsrändern hiervon und der Pupillen kann dabei manuell durch Markieren der Brillenfassung und Pupillen in dargestellten Bildern oder automatisch durch Bildverarbeitungsalgorithmen erfolgen, wie ebenfalls beschrieben. Ist das Auge, beispielsweise die Hornhaut des Auges, in den Seitenbildern nicht genau zu erkennen, weil es durch Brillenbügel verdeckt wird, kann auch zusätzlich eine Aufnahme ohne Brillenfassung eingeblendet werden, um eine Bestimmung der Pupillenlage zu ermöglichen. Zudem wird wie bereits oben erläutert eine Lage eines Augendrehpunktes abgeschätzt. Nach diesen Messungen ist also eine relative Lage des Augendrehpunktes zu der Brillenfassung sowie eine Form der Brillenfassung bekannt. Die Lage der Brillenfassung bestimmt dabei die Lage der Brillengläser und somit von Ebenen, in denen die Brillengläser liegen (auch als Glasebenen bezeichnet).

Mit diesen Daten können in der in den oben genannten europäischen Patentanmeldungen beschriebenen Weise dann Fern-Durchblickpunkte bestimmt werden.

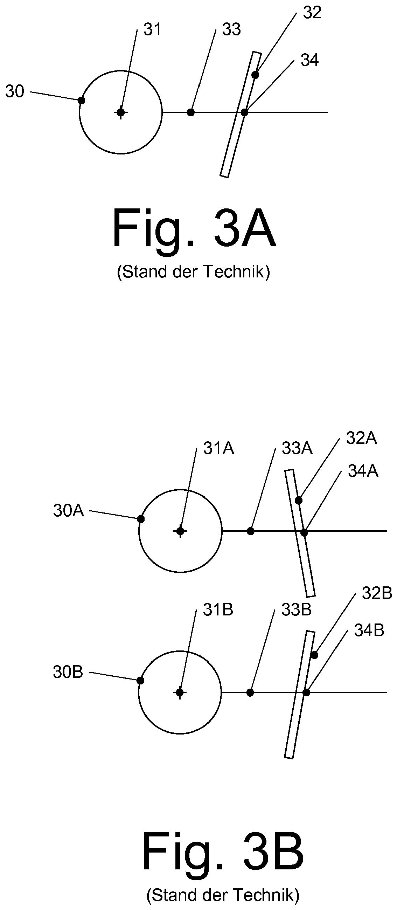

Diese Informationen werden dann bei dem beschriebenen Ausführungsbeispiel zur Bestimmung eines oder beider Nah-Durchblickpunkte verwendet. Zudem können wie in den europäischen Patentanmeldungen beschrieben Fern-Durckblickpunkte bestimmt werden. Dies ist in den Figuren 3A und 3B schematisch dargestellt. Die Figur 3A zeigt eine Lage eines Fern-Durchblickpunktes 34 für eine Blickrichtung 33 ausgehend von einem Augendrehpunkt 31 eines Auges 30 als Schnittpunkt der Blickrichtung 33 mit einem Brillenglas 32 in Seitenansicht. Die Fig. 3B zeigt eine Draufsicht für beide Augen, wobei die Bezugszeichen für das linke Auge jeweils ein angehängtes A aufweisen und die Bezugszeichen für das rechte Auge ein angehängtes B, und ansonsten den Bezugszeichen der Seitenansicht der Fig. 3A entsprechen.

Die Lage der Brillengläser 32 ergibt sich dabei aus den in den Bildaufnahmen identifizierten Brillenfassungen, insbesondere den Fassungsrändern hiervon.

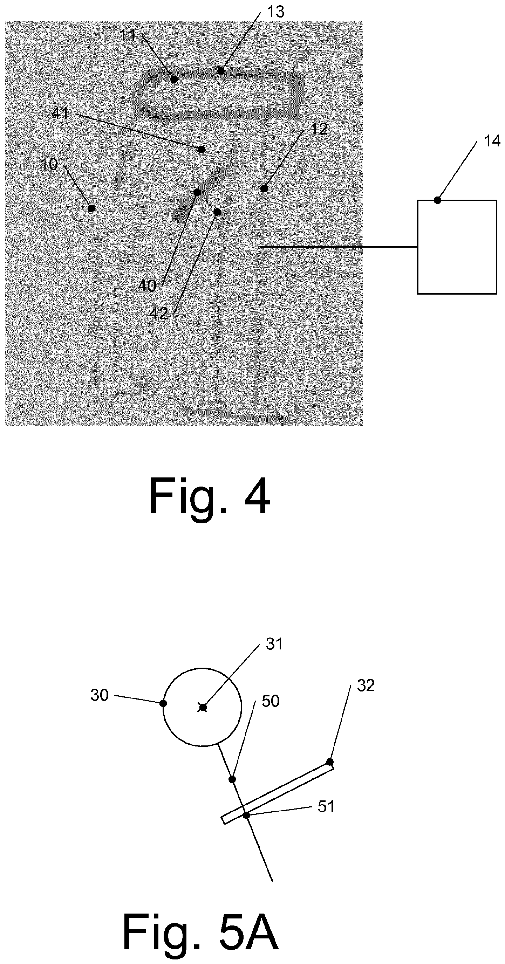

Die Fig. 4 zeigt eine Seitenansicht der Vorrichtung der Fig. 1 , welche zu einer erfindungsgemäßen Bestimmung eines Nah-Durchblickpunktes erweitert ist. Hier wird der Person 10 zusätzlich zu der Vorrichtung der Fig. 1 ein Nahblickziel 40 bereitgestellt, welches einen zu lesenden Text aufweist und auch als Lesetarget bezeichnet wird. Das Nahblickziel 40 ist dabei durch die Person 10 bewegbar, um es in eine Position zu bringen, die einer angenehmen Lesehaltung entspricht. Das Bezugszeichen 41 bezeichnet die Blickrichtung der Person 10 beim Lesen des Textes. Bei dem Ausführungsbeispiel der Fig. 4 ist das Nahblickziel 40 durch eine bewegliche Verbindung 42, die Sensoren umfasst, mit der Säule 12 verbunden. Mittels dieser Sensoren wird die Position des Nahblickziels 40 erfasst. Zudem werden durch die Kameraeinrichtung 13 in der in Fig. 4 dargestellten Position Bildaufnahmen des Kopfes 11 vorgenommen. In diesen Bildaufnahmen wird wiederum die Brillenfassung identifiziert. Dies kann gemeinsam mit der Identifizierung der Brillenfassung bei den Aufnahmen der Fig. 1 oder nach dieser erfolgen, wobei im letzteren Fall die in Fig. 1 bestimmte Brillenfassung als Basis verwendet wird. Als Randbedingungen können hier beispielsweise Modelle für Brillenfassungen dienen, wie sie in der europäischen Patentanmeldung 17 153 538.8 beschrieben sind.

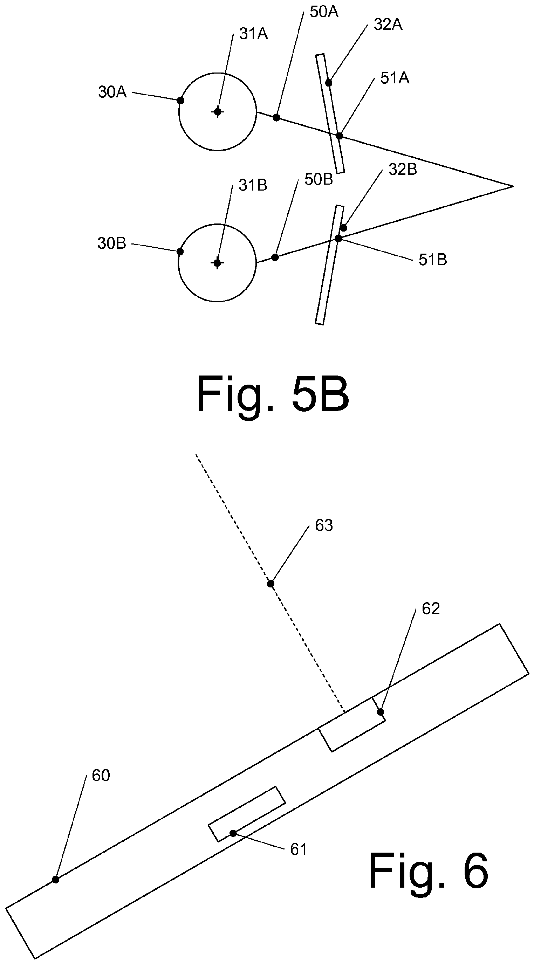

Die Recheneinrichtung 14 ist dann in Ergänzung zu der herkömmlichen Vorrichtung der Fig. 1 noch programmiert, Nah-Durchblickpunkte für beide Augen zu bestimmen. Hierbei wird die bei der Bestimmung der Fern-Durchblickpunkte in der Position der Fig. 1 ermittelte relative Lage des Augendrehpunktes zu der Fassung auf die nun bestimmte Lage der Brillenfassung übertragen und als Blickrichtung die Differenz der Position dieses Augendrehpunktes zu der Position des Nahblickziels 40 verwendet. So wird die Blickrichtung 41 bestimmt. Die Blickrichtung 41 ausgehend vom Augendrehpunkt schneidet die Brillengläser, deren Lagen durch die Lage der Brillenfassung definiert sind, dann in den Nah-Durchblickpunkten. Dies ist in den Figuren 5A und 5B schematisch dargestellt. Die Figuren 5A und 5B zeigen dabei Ansichten entsprechend den Figuren 3A und 3B für die Bestimmung von Nah-Durchblickpunkten 51 in Fig. 5A bzw. 51A, 51B in Fig. 5B. Die Blickrichtung 50 (50A, 50B für linkes und rechtes Auge) schneiden die Brillengläser 32A, 32B in den Nah-Durchblickpunkten 51A, 51B, welche sich wie aus einem Vergleich der Figuren 5A, 5B mit den Figuren 3A, 3B ersichtlich von den Fern-Durchblickpunkten unterscheiden.

Wie bereits eingangs erläutert kann alternativ zu der Positionsbestimmung des Nahblickziels 40 und die Bildaufnahme durch die Kameraeinrichtung 13 eine Bildaufnahme durch das Nahblickziel und eine Orientierungsbestimmung des Nahblickziels erfolgen. Ein entsprechendes Nahblickziel ist in Fig. 6 dargestellt.

Die Fig. 6 zeigt ein Nahblickziel 60, welches über eine eingebaute Kamera 62 verfügt. Die Kamera 62 weist eine Symmetrieachse 63 auf, welche der optischen Achse der Kamera 62 entspricht. Des Weiteren weist das Nahblickziel 60 einen Neigungssensor 61 auf. Das Nahblickziel 60 ist dabei ein Tablet-Computer oder Smartphone, welche üblicherweise über Neigungssensoren sowie eingebaute Kameras verfügen. Bei der Ausführungsform der Fig. 6 wird mittels der Kamera 62 ein Bild des Kopfes der Person aufgenommen, während sie das Nahblickziel 60 betrachtet. Aus der Orientierung des Nahblickziels 60 wie durch den Neigungssensor 61 bestimmt und somit der Orientierung der Symmetrieachse 63, den Eigenschaften der Kamera 62 (Bildwinkel und Auflösung) sowie dem aufgenommenen Bild kann dann wie beschrieben die Blickrichtung der Person bestimmt werden, und auf Basis der Blickrichtung dann wie oben beschrieben die Nahdurchblickpunkte.

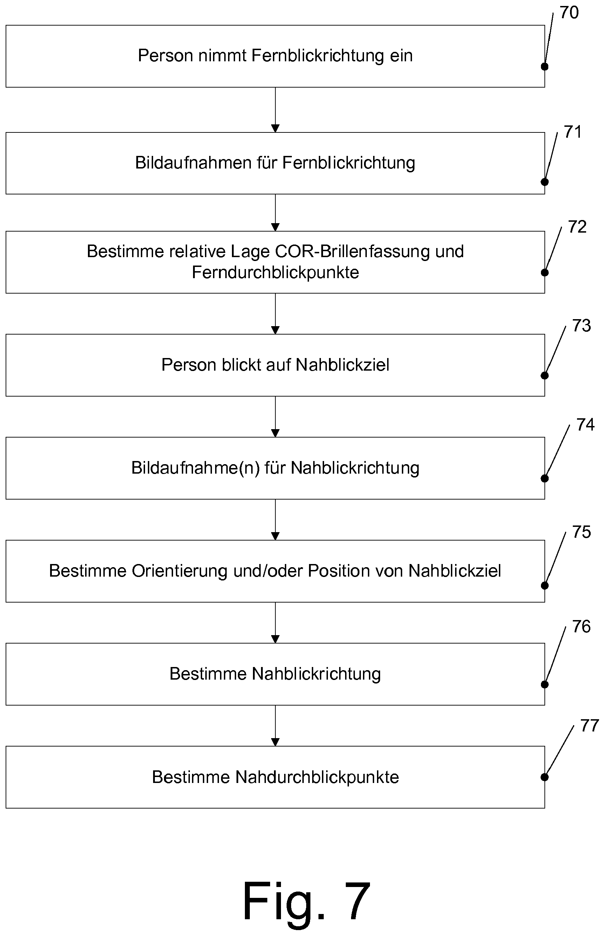

Die Fig. 7 zeigt ein Flussdiagramm eines Verfahrens gemäß einem Ausführungsbeispiel. Das Verfahren der Fig. 7 wird zur besseren Veranschaulichung unter Bezugnahme auf die in den Figuren 1 bis 6 dargestellten Vorrichtungen erläutert.

In Schritt 70 nimmt eine zu untersuchende Person eine Fernblickrichtung ein, beispielsweise die in den Figuren 1 und 2 dargestellte Position, bei welchen der Benutzer gerade horizontal nach vorne blickt. In Schritt 71 erfolgen dann Bildaufnahmen für die Fernblickrichtung, im Falle der Figuren 1 und 2 mittels der Kameraeinrichtung 13. Aus diesen Bildaufnahmen wird wie bereits beschrieben in Schritt 72 die Lage einer Brillenfassung, die Lage von Pupillen und daraus die relative Lage des Augendrehpunktes (COR; Center of Rotation) zu der Brillenfassung bestimmt. Zudem werden in Schritt 72 Fern-Durchblickpunkte wie in den Figuren 3A und 3B erläutert bestimmt.

In Schritt 73 blickt die Person dann auf ein Nahblickziel, wie dies in der Fig. 4 für das Nahblickziel 40 gezeigt ist, oder auf das Nahblickziel 60 der Fig. 6. In Schritt 74 erfolgen dann eine oder mehrere Bildaufnahmen für die Nahblickrichtung, beispielsweise mehrere Bildaufnahmen mittels der Kameraeinrichtung 13 der Fig. 4 oder eine Bildaufnahme mit der Kamera 62 des Nahblickziels 60 der Fig. 6. In Schritt 75 werden Orientierung (beispielsweise mit dem Lagesensor 61) und/oder Position (beispielsweise mit den Sensoren der Verbindung 42 der Fig. 4) des Nahblickziels bestimmt. Aus den so ermittelten Daten wird wie beschrieben in Schritt 76 die Nahblickrichtung bestimmt. Optional kann in Schritt 76 zusätzlich eine Korrektur der Nahblickrichtung durch einen Benutzer wie einen Optiker erfolgen. Diese Korrektur kann in verschiedenen Richtungen erfolgen, beispielsweise falls die Person zum Zeitpunkt der Bildaufnahme keine natürliche Kopfhaltung eingenommen hat. In Schritt 77 werden dann Nah-Durchblickpunkte bestimmt, indem die Nahblickrichtung ausgehend von dem Augendrehpunkt mit den Brillengläsern geschnitten wird, wie dies in den Figuren 5A und 5B gezeigt ist.

Die Kameraeinrichtung 13 weist dabei intrinsisch und extrinsisch kalibrierte Kameras auf, d. h. die Lage der Kameras zueinander sowie deren Richtungen und Eigenschaften sind bekannt, und beispielsweise Bildfeldverzeichnungen können herausgerechnet werden.

## Patentansprüche

1. Verfahren zum Bestimmen eines Nah-Durchblickpunktes (51), umfassend:
Aufnehmen eines Bildes eines Kopfes mit Augen einer Person, wobei das Aufnehmen des Bildes mit einer in einem Nahblickziel (40; 60) eingebauten Kamera (62) erfolgt, während die Person auf das Nahblickziel (40; 60) blickt,
wobei das Nahblickziel (40; 60) beweglich ist, und das Verfahren weiter umfasst:
Bestimmen einer Orientierung des Nahblickziels (40; 60),
Bestimmen einer Blickrichtung der Person beim Blick auf das Nahblickziel (40; 60) auf Basis des Bildes, **dadurch gekennzeichnet, dass**
das Bestimmen des Nah-Durchblickpunktes auf Basis des Bildes und auf Basis der Blickrichtung erfolgt,
wobei das Bestimmen der Blickrichtung umfasst:
ein Bestimmen einer Position einer Pupille eines Auges der Augen in dem Bild,
Bestimmen einer Richtung der Pupille relativ zu einer Symmetrieachse der Kamera basierend auf einem Bildwinkel, einer Auflösung der Kamera (62) und der Position der Pupille des einen Auges in dem Bild,
Bestimmen einer Richtung der Symmetrieachse basierend auf der Orientierung des Nahblickziels (40; 60),
Bestimmen der Blickrichtung basierend auf der Richtung der Pupille und der Richtung der Symmetrieachse,
und wobei das Bestimmen des Nah-Durchblickpunktes ferner auf einer der folgenden Alternativen basiert:
i) wenn die Person eine Brillenfassung trägt:
Identifizieren und Bestimmen einer Lage der Brillenfassung in dem aufgenommenen Bild, wobei das Bestimmen des Nah-Durchblickpunktes ferner auf der Lage der Brillenfassung basiert,
ii) wenn die Person keine Brillenfassung trägt:
Bestimmen des Nah-Durchblickpunktes auf Basis der bestimmten Blickrichtung und eines Modells des Kopfes, an welchen ein Modell einer Brillenfassung angepasst ist.

2. Verfahren nach Anspruch 1, wobei das Verfahren ferner umfasst:
eine intrinsische Kalibrierung der Kamera (62), um Bildfeldverzeichnungen zu korrigieren und wobei das Bestimmen des Nah-Durchblickpunktes (51) ferner auf der intrinsischen Kalibrierung basiert.

3. Verfahren nach Anspruch 1 oder 2, weiter umfassend:
Bestimmen einer relativen Lage einer Brillenfassung zu einem Augendrehpunkt der Person,
wobei der Nah-Durchblickpunkt durch Ermitteln eines Schnittpunktes einer Linie in Blickrichtung ausgehend von dem Augendrehpunkt mit einer durch die Lage der Brillenfassung definierten Lage einer Brillenglasebene (32) bestimmt wird.

4. Verfahren nach Anspruch 3, wobei das Bestimmen der relativen Lage der Brillenfassung zu dem Augendrehpunkt auf Basis weiterer Bildaufnahmen erfolgt.

5. Verfahren nach Anspruch 4, weiter umfassend Bestimmen eines Fern-Durchblickpunktes auf Basis der weiteren Bildaufnahmen.

6. Verfahren nach Anspruch 4 oder 5, weiter umfassend ein Bestimmen der Lage der Brillenfassung auf Basis der weiteren Bildaufnahmen.

7. Verfahren nach Anspruch 6, weiter umfassend ein Bestimmen einer Lage der Brillenfassung in dem aufgenommen Bild, wobei das Bestimmen des Nah-Durchblickpunktes auf Basis der Lage der Brillenfassung in dem aufgenommenen Bild erfolgt.

8. Verfahren nach Anspruch 7, wobei das Bestimmen der Lage der Brillenfassung in dem aufgenommenen Bild auf Basis des Bestimmens der Lage der Brillenfassung in den weiteren Bildaufnahmen oder gleichzeitig mit dem Bestimmen der Lage der Brillenfassung in den weiteren Bildaufnahmen erfolgt.

9. Verfahren nach einem der Ansprüche 5-8, wobei das Aufnehmen des Bildes mit einer Bildaufnahmeeinrichtung (13; 62) erfolgt, wobei die weiteren Bildaufnahmen mit der gleichen Bildaufnahmeeinrichtung (13; 62) erfolgen wie das Aufnehmen des Bildes.

10. Verfahren nach einem der Ansprüche 1-9, wobei das Verfahren ein Korrigieren der bestimmten Blickrichtung und/oder ein Korrigieren des bestimmten Nah-Durchblickpunktes umfasst.

11. Verfahren nach einem der Ansprüche 1-10, wobei das Bestimmen des Nah-Durchblickpunktes auf Basis der bestimmten Blickrichtung und eines Modells des Kopfes, an welchen ein Modell einer Brillenfassung angepasst ist, erfolgt.

12. Computerprogramm mit einem Programmcode, welcher, wenn er auf einem Prozessor ausgeführt wird, bewirkt, dass das Verfahren nach einem der Ansprüche 1-11 durchgeführt wird.

13. Vorrichtung zum Bestimmen eines Nahdurchblickpunktes (51), umfassend:
eine Recheneinrichtung (14),
ein Nahblickziel (40; 60), welches beweglich ist und eine Bildaufnahmeeinrichtung (62) und eine Positionserfassungseinrichtung (61) umfasst,
wobei die Bildaufnahmeeinrichtung (13; 62) eingerichtet ist, ein Bild eines Kopfes mit Augen einer Person aufzunehmen, während die Person auf das Nahblickziel (40; 60) blickt,
wobei die Positionserfassungseinrichtung (42; 61) zum Bestimmen einer Orientierung des Nahblickziels (40; 60) eingerichtet ist,
wobei die Recheneinrichtung (14) zum Bestimmen einer Blickrichtung der Person beim Blick auf das Nahblickziel auf Basis des Bildes eingerichtet ist, **dadurch gekennzeichnet, dass**
die Recheneinrichtung weiter eingerichtet ist zum Bestimmen des Nah-Durchblickpunktes (51) auf Basis des Bildes und auf Basis der Blickrichtung, wobei das Bestimmen der Blickrichtung umfasst:
ein Bestimmen einer Position einer Pupille eines Auges der Augen in dem Bild,
Bestimmen einer Richtung der Pupille relativ zu einer Symmetrieachse der Kamera basierend auf einem Bildwinkel, einer Auflösung der Kamera (62) und der Position der Pupille des einen Auges in dem Bild,
Bestimmen einer Richtung der Symmetrieachse basierend auf der Orientierung des Nahblickziels (40; 60),
Bestimmen der Blickrichtung basierend auf der Richtung der Pupille und der Richtung der Symmetrieachse,
wobei das Bestimmen des Nah-Durchblickpunktes ferner auf einer der folgenden Alternativen basiert:
i) wenn die Person eine Brillenfassung trägt:
Identifizieren und Bestimmen einer Lage der Brillenfassung in dem aufgenommenen Bild, wobei das Bestimmen des Nah-Durchblickpunktes ferner auf der Lage der Brillenfassung basiert,
ii) wenn die Person keine Brillenfassung trägt:
Bestimmen des Nah-Durchblickpunktes auf Basis der bestimmten Blickrichtung und eines Modells des Kopfes, an welchen ein Modell einer Brillenfassung angepasst ist.

14. Vorrichtung nach Anspruch 13, wobei die Recheneinrichtung weiter eingerichtet ist, eine intrinsische Kalibrierung der Kamera (62), um Bildfeldverzeichnungen zu korrigieren, zu berücksichtigen und wobei das Bestimmen des Nah-Durchblickpunktes (51) ferner auf der intrinsischen Kalibrierung basiert.

## Claims

1. Method for determining a near visual point (51), comprising:
recording an image of a head with the eyes of a person, wherein the image is recorded with a camera (62) installed at a near-vision target (40; 60) while the person gazes at the near-vision target (40; 60),
wherein the near-vision target (40; 60) is movable and the method further comprises:
determining an orientation of the near-vision target (40; 60),
determining a viewing direction of the person when gazing at the near-vision target (40; 60) on the basis of the image, **characterized in that**
the near visual point is determined on the basis of the image and on the basis of the viewing direction, wherein determining the viewing direction comprises:
determining a position of a pupil of an eye of the eyes in the image,
determining a direction of the pupil relative to an axis of symmetry of the camera on the basis of an image angle, a resolution of the camera (62) and the position of the pupil of the one eye in the image, determining a direction of the axis of symmetry on the basis of the orientation of the near-vision target (40; 60),
determining the viewing direction on the basis of the direction of the pupil and the direction of the axis of symmetry,
and wherein determining the near visual point is further based on one of the following alternatives:
i) if the person is wearing the spectacle frame: identifying and determining a position and orientation of the spectacle frame in the recorded image, wherein the determination of the near visual point is further based on the position and orientation of the spectacle frame,
ii) if the person is wearing no spectacle frame: determining the near visual point on the basis of the determined viewing direction and a model of the head, to which a model of a spectacle frame is fitted.

2. Method according to Claim 1, wherein the method further comprises:
an intrinsic calibration of the camera (62) in order to correct image field distortions, wherein the determination of the near visual point (51) is further based on the intrinsic calibration.

3. Method according to Claim 1 or 2, further comprising:
determining a relative position and orientation of a spectacle frame with respect to a centre of rotation of the eye of the person,
wherein the near visual point is determined by ascertaining a point of intersection of a line in the viewing direction, proceeding from the centre of rotation of the eye, with a defined position and orientation of a spectacle lens plane (32) defined by the position and orientation of the spectacle frame.

4. Method according to Claim 3, wherein the relative position and orientation of the spectacle frame with respect to the centre of rotation of the eye is determined on the basis of further image recordings.

5. Method according to Claim 4, further comprising determining a distance visual point on the basis of the further image recordings.

6. Method according to Claim 4 or 5, further comprising determining the position and orientation of the spectacle frame on the basis of the further image recordings.

7. Method according to Claim 6, further comprising determining a position and orientation of the spectacle frame in the recorded image, wherein the near visual point is determined on the basis of the position and orientation of the spectacle frame in the recorded image.

8. Method according to Claim 7, wherein determining the position and orientation of the spectacle frame in the recorded image is implemented on the basis of the determination of the position and orientation of the spectacle frame in the further image recordings or at the same time as the determination of the position and orientation of the spectacle frame in the further image recordings.

9. Method according to any one of Claims 5-8, wherein the image is recorded by an image recording device (13; 62), wherein the further image recordings are implemented by the same image recording device (13; 62) used for recording of the image.

10. Method according to any one of Claims 1-9, wherein the method comprises correcting the determined viewing direction and/or correcting the determined near visual point.

11. Method according to any one of Claims 1-10, wherein the near visual point is determined on the basis of the determined viewing direction and on the basis of a model of the head, to which a model of a spectacle frame is fitted.

12. Computer program comprising program code which, when executed on a processor, causes the method according to any one of Claims 1-11 to be carried out.

13. Apparatus for determining a near visual point (51), comprising:
a computing device (14),
a near-vision target (40; 60), which is movable and which comprises an image recording device (62) and a position detection device (61),
wherein the image recording device (13; 62) is configured to record an image of a head with eyes of a person while the person gazes on the near-vision target (40; 60),
wherein the position detection device (42; 61) is configured to determine an orientation of the near-vision target (40; 60),
wherein the computing device (14) is configured to determine, on the basis of the image, a viewing direction of the person gazing on the near-vision target, **characterized in that**
the computing device is further configured to determine the near visual point (51) on the basis of the image and on the basis of viewing direction, wherein
determining the viewing direction comprises:
determining a position of a pupil of an eye of the eyes in the image,
determining a direction of the pupil relative to an axis of symmetry of the camera on the basis of an image angle, a resolution of the camera (62) and the position of the pupil of the one eye in the image, determining a direction of the axis of symmetry on the basis of the orientation of the near-vision target (40; 60),
determining the viewing direction on the basis of the direction of the pupil and the direction of the axis of symmetry,
wherein determining the near visual point is further based on one of the following alternatives:
i) if the person is wearing a spectacle frame: identifying and determining a position and orientation of the spectacle frame in the recorded image, wherein the determination of the near visual point is further based on the position and orientation of the spectacle frame,
ii) if the person is wearing no spectacle frame: determining the near visual point on the basis of the determined viewing direction and a model of the head, to which a model of a spectacle frame is fitted.

14. Apparatus according to Claim 13, wherein the computing device is further configured to take account of an intrinsic calibration of the camera (62) in order to correct image field distortions and wherein the determination of the near visual point (51) is further based on the intrinsic calibration.

## Revendications

1. Procédé de détermination d'un point de vision de près (51), comprenant :
enregistrement d'une image d'une tête avec les yeux d'une personne, l'enregistrement de l'image étant réalisé avec une caméra (62) incorporée dans une cible de vision de près (40 ; 60) pendant que la personne regarde la cible de vision de près (40 ; 60),
la cible de vision de près (40 ; 60) étant mobile et le procédé comprenant en outre :
détermination d'une orientation de la cible de vision de près (40 ; 60),
détermination d'une direction du regard de la personne lorsqu'elle regarde la cible de vision de près (40 ; 60) en se basant sur l'image, **caractérisé en ce que** la détermination du point de vision de près s'effectue en se basant sur l'image et en se basant sur la direction du regard,
la détermination de la direction du regard comprenant :
une détermination d'une position d'une pupille d'un œil des yeux dans l'image,
détermination d'une direction de la pupille par rapport à un axe de symétrie de la caméra en se basant sur un angle de vision, une résolution de la caméra (62) et la position de la pupille d'un œil dans l'image,
détermination d'une direction de l'axe de symétrie en se basant sur l'orientation de la cible de vision de près (40 ; 60),
détermination de la direction du regard en se basant sur la direction de la pupille et la direction de l'axe de symétrie,
et la détermination du point de vision de près se basant en outre sur l'une des alternatives suivantes :
i) lorsque la personne porte une monture de lunettes : identification et détermination d'une position de la monture de lunettes dans l'image enregistrée, la détermination du point de vision de près se basant en outre sur la position de la monture de lunettes,
ii) lorsque la personne ne porte pas de monture de lunettes :
détermination du point de vision de près en se basant sur la direction du regard déterminée et un modèle de la tête auquel est adapté un modèle d'une monture de lunettes.

2. Procédé selon la revendication 1, le procédé comprenant en outre :
un étalonnage intrinsèque de la caméra (62) visant à corriger les distorsions du champ d'image et la détermination du point de vision de près (51) se basant en outre sur l'étalonnage intrinsèque.

3. Procédé selon la revendication 1 ou 2, le procédé comprenant en outre :
détermination d'une position relative d'une monture de lunettes par rapport à un centre de rotation de l'œil de la personne,
le point de vision de près étant déterminé en établissant un point d'intersection d'une ligne dans la direction du regard partant du centre de rotation de l'œil avec la position d'un plan de verre de lunettes (32) défini par la position de la monture de lunettes.

4. Procédé selon la revendication 3, la détermination de la position relative de la monture de lunettes par rapport au centre de rotation de l'œil s'effectuant en se basant sur des enregistrements d'image supplémentaires.

5. Procédé selon la revendication 4, comprenant en outre la détermination d'un point de vision de loin en se basant sur les enregistrements d'image supplémentaires.

6. Procédé selon la revendication 4 ou 5, comprenant en outre une détermination de la position de la monture de lunettes en se basant sur les enregistrements d'image supplémentaires.

7. Procédé selon la revendication 6, comprenant en outre une détermination d'une position de la monture de lunettes dans l'image enregistrée, la détermination du point de vision de près s'effectuant en se basant sur la position de la monture de lunettes dans l'image enregistrée.

8. Procédé selon la revendication 7, la détermination de la position de la monture de lunettes dans l'image enregistrée s'effectuant en se basant sur la détermination de la position de la monture de lunettes dans les enregistrements d'image supplémentaires ou simultanément avec la détermination de la position de la monture de lunettes dans les enregistrements d'image supplémentaires.

9. Procédé selon l'une des revendications 5 à 8, l'enregistrement de l'image étant effectué avec un dispositif d'enregistrement d'images (13 ; 62), les enregistrements d'image supplémentaires s'effectuant avec le même dispositif d'enregistrement d'images (13 ; 62) que l'enregistrement de l'image.

10. Procédé selon l'une des revendications 1 à 9, le procédé comprenant une correction de la direction du regard déterminée et/ou une correction du point de vision de près déterminé.

11. Procédé selon l'une des revendications 1 à 10, la détermination du point de vision de près s'effectuant en se basant sur la direction du regard déterminée et un modèle de la tête auquel est adapté un modèle d'une monture de lunettes.

12. Programme informatique comprenant un code de programme qui, lorsqu'il est exécuté sur un processeur, a pour effet que le procédé selon l'une des revendications 1 à 11 est mis en œuvre.

13. Arrangement de détermination d'un point de vision de près (51), comprenant :
un dispositif de calcul (14),
une cible de vision de près (40 ; 60), laquelle est mobile et comporte un dispositif d'enregistrement d'image (62) et un dispositif de détection de position (61),
le dispositif d'enregistrement d'image (13 ; 62) étant conçu pour enregistrer une image d'une tête avec les yeux d'une personne pendant que la personne regarde la cible de vision de près (40 ; 60),
le dispositif de détection de position (42 ; 61) étant conçu pour déterminer une orientation de la cible de vision de près (40 ; 60),
le dispositif de calcul (14) étant conçu pour déterminer une direction du regard de la personne lorsqu'elle regarde la cible de vision de près en se basant sur l'image, **caractérisé en ce que**
le dispositif de calcul est en outre conçu pour déterminer le point de vision de près (51) en se basant sur l'image et en se basant sur la direction du regard, la détermination de la direction du regard comprenant :
une détermination d'une position d'une pupille d'un œil des yeux dans l'image,
détermination d'une direction de la pupille par rapport à un axe de symétrie de la caméra en se basant sur un angle de vision, une résolution de la caméra (62) et la position de la pupille d'un œil dans l'image,
détermination d'une direction de l'axe de symétrie en se basant sur l'orientation de la cible de vision de près (40 ; 60),
détermination de la direction du regard en se basant sur la direction de la pupille et la direction de l'axe de symétrie,
la détermination du point de vision de près se basant en outre sur l'une des alternatives suivantes :
i) lorsque la personne porte une monture de lunettes : identification et détermination d'une position de la monture de lunettes dans l'image enregistrée, la détermination du point de vision de près se basant en outre sur la position de la monture de lunettes,
ii) lorsque la personne ne porte pas de monture de lunettes :
détermination du point de vision de près en se basant sur la direction du regard déterminée et un modèle de la tête auquel est adapté un modèle d'une monture de lunettes.

14. Arrangement selon la revendication 13, le dispositif de calcul étant en outre conçu pour tenir compte d'un étalonnage intrinsèque de la caméra (62) visant à corriger les distorsions du champ d'image et la détermination du point de vision de près (51) se basant en outre sur l'étalonnage intrinsèque.
